# EUROPEAN PATENT SPECIFICATION

(11) **EP 2 134 876 B1**
(45) Date of publication and mention of the grant of the patent: **07.05.2014**
(21) Application number: 06784044.7
(22) Date of filing: 06.09.2006
(51) Int. Cl.: C12Q 1/68

(54) **DETERMINING A PREDISPOSITION TO BREAST CANCER BY IDENTIFICATION OF GENOTYPE COMBINATIONS OF SPECIFIC VARIANTS OF THE GENES CYP1B1, BRCA2 AND CHEK2**
BESTIMMUNG EINER VERANLAGUNG FÜR BRUSTKREBS DURCH IDENTIFIZIERUNG VON GENOTYPKOMBINATIONEN SPEZIFISCHER VARIANTEN DER GENE CYP1B1, BRCA2 UND CHEK2
DÉTERMINATION DE LA PRÉDISPOSITION AU CANCER Du SEIN PAR L'IDENTIFICATION DE COMBINAISONS GÉNOTYPIQUES DE VARIANTS SPÉCIFIQUES DES GÈNES CYP1B1, BRCA2 ET CHEK2

(30) Priority: 22.06.2006 WO PCT/PL2006/000040
(43) Date of publication of application: 23.12.2009
(73) Proprietor: Pomorski Uniwersytet Medyczny, 70-204 Szczecin (PL); Lubinski, Jan, PL-71-253 Szczecin (PL); Cybulski, Cezary, PL-72-005 Przeclaw (PL); Debniak, Tadeusz, 70-736 Szczecin (PL); Kurzawski, Grzegorz, 71-671 Szczecin (PL); Suchy, Janina, 71-004 Szczecin (PL); Serrano-Fernandez, Pablo, 70-115 Szczecin (PL); Matyjasik, Joanna, 70-010 Police (PL); Gorski, Bohdan, 70-115 Szczecin (PL)
(72) Inventor: LUBINSKI, Jan, PL-71-253 Szczecin (PL); CYBULSKI, Cezary, PL-72-005 Przeclaw (PL); DEBNIAK, Tadeusz, PL-70-736 Szczecin (PL); KURZAWSKI, Grzegorz, PL-71-671 Szczecin (PL); SUCHY, Janina, PL-71-004 Szczecin (PL); SERRANO-FERNANDEZ, Pablo, PL-70-115 Szczecin (PL); MATYJASIK, Joanna, PL-72-010 Police (PL); GORSKI, Bohdan, PL-70-115 Szczecin (PL)
(74) Representative: Witek, Rafal
(86) International application number: PCT/PL2006/000062
(87) International publication number: WO 2007/148997

(56) References cited:
- WO-A-02/30951
- US-A1- 6 033 857
- WANQING WEN ET AL: "cytochrome p450 1b1 and catechol-o-methyltransferase genetic polymorphisms and breast cancer risk in chinese women: results from shanghai breast cancer study and a meta-analysis" CANCER EPIDEMIOLOGY, BIOMARKERS AND PREVENTION, AMERICAN ASSOCIATION FOR CANCER RESEARCH,, US, vol. 14, no. 2, February 2005 (2005-02), pages 329-335, XP002412157 ISSN: 1055-9965
- SASAKI M ET AL: "polymorphisms of the cyp1b1 gene as risk factors for human renal cell cancer" CLINICAL CANCER RESEARCH, THE AMERICAN ASSOCIATION FOR CANCER RESEARCH, US, vol. 10, 15 March 2004 (2004-03-15), pages 2015-2019, XP002412158 ISSN: 1078-0432
- DONG X ET AL: "Mutations in CHEK2 associated with prostate cancer risk" AMERICAN JOURNAL OF HUMAN GENETICS, AMERICAN SOCIETY OF HUMAN GENETICS, CHICAGO, IL, US, vol. 72, no. 2, February 2003 (2003-02), pages 270-280, XP002339599 ISSN: 0002-9297
- DONG JIN-TANG: "prevalent mutations in prostate cancer" J. CELL. BIOCHEMISTRY, vol. 97, 15 February 2006 (2006-02-15), pages 433-447, XP002425412

## Description

### FIELD OF THE INVENTION

Mode and composition for detection of inherited predisposition to cancers of various sites and the use of particular genotype combinations of germline variants within CYP1B1, CHEK2 and BRCA2 genes for diagnosing of such predisposition are subject of invention. Generally, the invention concerns the new diagnostic method. Subject of invention allow to synthesize DNA and identification of genomic abnormalities which are correlated with increased genetic predisposition to cancers of various organs, which is not just the sum of the effects of the single variants considered.

Several documents are cited throughout the text of this specification.

### BACKGROUND OF THE INVENTION

### CYP1B1

CYP1B1 is one of the major enzymes of cytochrome P450 involved in carcinogenic hydroxylation (Hayes et al., Proc. Natl. Acad. Sci. USA 93:9776-81,1996; Shimada et al., Cancer Res. 56:2979-84,1996; Spink et al., J. Steroid. Biochem. Mol. Biol. 51:251-8,1994). The hydroxylation activity of CYP1B1 metabolises estradiol to 4-hydroxyestradiol, which is a catechol estrogen that has been shown to induce depurination of DNA that leads to DNA mutations (Bailey et al., Cancer Res. 58:5038-41,1998; Chakravarti et al., Oncogene 20:7945-53,2001; Spink et al., J. Steroid. Biochem. Mol. Biol. 2/3:223-32,1997). In addition to estrogens, CYP1B1 also activates a range of chemically diverse procarcinogens including benzo[a]pyrene (Rylander-Rudqvist et al., Carcinogenesis 24:1533-9,2003; Shimada et al., Cancer Res. 56:2979-84,1996,20).

Several studies indicate that some variants of CYP1B1 are showing unusually high carcinogenic hydroxylation activities (Hanna et al., Cancer Res. 60:3440-4,2000; Shimada et al., Cancer Res. 56:2979-84,1996; Shimada et al., Carcinogenesis 20/8:1607-13,1999). The potential relationship between various single nucleotide polymorphism sites (SNPs) present in the human CYP1B1 gene and the incidence of different cancer types has been suggested (Fritzsche et al., Pharmacogenetics 9:405-8,1999; Goodman et al., Cancer Epidemiol Biomarkers Prev. 10(3):209-16,2001).

International patent application WO 02/04683 is showing the mode of identification of persons with increased risk of estrogen-dependent cancers, particularly cancers of the breast, wherein CYP1B1, CYP1A1, COMT and GSTM1 genotypes are examined. Among SNPs occurring on CYP1B1 gene, only codons 432 and 453 are used in above application. Patent application US 20040002071 contains the mode of assessment of breast cancer risk by examination of at least two genes. Among alleles increasing probability of cancer, there are two CYP1B1 alleles containing G or C at nucleotide position 1294 (GeneBank accession No U 56438) which corresponds to codon 432.

CYP1B1 355T/T variant is positively associated with development of a prostate cancer (Tanaka et al., BBRC 296:820-6,2002) with more aggressive clinical characteristics (Cicek et al., Cancer Epidemiol. Biomarkers Prev. 14(9):2173-7,2005), and is also associated with kidney cancer (Sasaki et al., Clinical Cancer Res. 10:2015-9,2004) and early onset breast and laryngeal cancer (Jaworoska et al., Breast Cancer Res. Treat. 97(2):215-9,2006). The predisposing effects of the 355T/T variant for cancer in multiple sites has also been object of a patent: US 60/693,149.

Some reports also show association of a whole haplotype of polymorphic SNPs of the CYP1B1 gene with cancer. Haplotype G-G-C-⁴⁸G¹¹⁹ was proved to be associated with increased risk of prostate cancer and haplotypes T-A-T-G⁴⁸-T¹¹⁹ and G⁴⁸T¹¹⁹-C⁴³² A⁴⁵³ are associated with decreased risk of prostate cancer (Chang et al., British J. Cancer 89:1524-9,2003). Haplotype 355T- 4326C is positively associated with prostate cancer among men with high aggressive disease (Cicek et al., Cancer Epidemiol. Biomarkers Prev. 14(9):2173-7,2005).

### CHEK2

CHEK2, (also known as"CHK2" [MIM 604373]) is a checkpoint kinase, which is activated in response to DNA damage. Activated CHEK2 phosphorylates BRCA1 and TP53 proteins, regulating tumor suppressor function of these proteins and thus prevents the proliferation of the affected cell (Matsuoka et al., Proc. Natl. Acad. Sci. USA, 97:10389-94,2000; Chaturvedi et al., Oncogene 18: 4047-54,1999; Ahn et al., Cancer Res. 60:5934-6,2000; Falck et al., Nature 410:842-7,2001; Chehab et al., Genes Dev. 14: 278-288,2000; Shieh et al., Genes Dev. 14: 289-300,2000; Lee et al., Nature 404: 201-4,2000.).

In Poland there are mainly four polymorphic variants of CHEK2, which are present in 5.7% of the population when taken together; three of these (1100delC, de15395, and IVS2+1G>A) are relatively rare and result in premature protein truncation. The third is a common missense variant (I157T) that results in aminoacid substitution: an isoleucine for a threonine.

All four variants have been found to be associated with increased predisposition to breast cancer (Cybulski et al. Clin. Cancer Res., 12(16):4832-5, 2006; Cybulski et al., Breast Cancer Res. Treat. electronic publication ahead of print,2006, Weischer et al., J. Clin. Oncol. electronic publication ahead of print,2006). The I157T variant also predisposes to colorectal cancer (Kilpivaara et al., J. Med. Genet. 43(7):e34, 2006), certain types of ovarian cancer (Szymanska-Pasternak et al., Gynecol. Oncol. electronic publication ahead of print,2006) and prostate cancer (Seppala et al. , Br. J. Cancer, 89: 1966-70,2003). The 1100delC variant also increases the risk of prostate cancer (Seppala et al., Br. J. Cancer, 89: 1966-70,2003). Although the full range of cancer types associated with inactivating mutations of CHEK2 has not yet been determined, these mutations have proved to be involved in multisite cancer at least for the Polish population and others.

Patent WO2005068659 claims the identification of the protein truncating variants IVS2+1G>A and 1100delC and the missense variant I157T of CHEK2 as indicators of increased colorectal and prostate cancer risk.

### BRCA2

The breast cancer susceptibility gene 2 (BRCA2) is a highly polymorphic gene known to play a major role in DNA-repair and control of cell proliferation. BRCA2 gene promotes homologous recombination, which comprises one major pathway of DNA double-strand break repair and thus may function to maintain genomic integrity and suppress tumor development in proliferating cells (Sharan et al., Nature 386:804-810, 1997; Xia et al., Proc. Nat. Acad. Sci. 98:8644-49,2001.; Moynahan et al., Mol. Cell 7: 263-272, 2001., Davies et al., Mol. Cell 7: 273-282,2001.).

Several variants of BRCA2 such as 6174delT, A1342C, 999del5, 185delAG, 5382insC, 6174delT, 8765delAG, and others have been demonstrated to increase breast cancer risk (Friedman et al., Am. J. Hum. Genet. 63:1224-1227, 1998; Healey et al., Nat. Genet. 26(3):362-4, 2000; Tulinius et al., J. Med. Genet. 39(7):457-62,2002; Tonin et al., Am. J. Hum. Genet. 63(5):1341-51,1998) and also other types of cancer like stomach, ovaries, prostate, pancreas, female genital tract, thyroid or colorectal cancer (Risch et al., Am. J. Hum. Genet. 68(3):700-10,2001; Thorlacius et al., Nat. Genet. 13(1):117-9,1996). Particularly interesting for the Polish population is BRCA2 variant C5972T, which increases the risk of developing breast cancer at young age (Gorski et al., Breast Cancer Res. 7(6):R1023-7,2005). Screening panels for several BRCA2 variants designed for the evaluation of breast and ovarian cancer have already been subject of international and US patents (WO9915701; WO9915704; WO9928506; WO9909164; WO03068054; US6033857; US2004115717; US2006154272), including BRCA2 C5972T variant which is comprised in the panel claimed by the US patent US6033857. Also a splice variant of BRCA2 has been claimed as a potential marker for breast and ovarian cancer risk in the US Patent US2002031785.

### Genetic interactions

The combined use of different genes to achieve a specific aim is a common subject of patent claims in biotechnology. However, there is little precedent of the use of genetic interactions for diagnostic and preventive purposes in medical genetics. The detection of some mutations of the RAD51 gene has been claimed as a method for diagnosing genetic predisposition or susceptibility to breast cancer among BRCA1 and BRCA2 mutation carriers (WO0118254), thus pointing towards a possible interaction between the BRCA1 and BRCA2 genes with the RAD51 gene. Patent W02006044933 claims the use of compounds which disrupt the interaction between RAD51 and BRCA2 in the context of cancer therapy.

Interactions between variants of the genes CYP1B1, CHEK2 and BRCA2 have neither been published in scientific literature nor been object of a patent application.

Diagnostic methods to identify increased predisposition to cancer of various sites is a major topic in medical genetics. With the advent of new technologies and extended databases, the focus is drifting from particular genetic variants associated with increased cancer risk to specific interactions between these genes which modulate that risk in a non-linear way. This invention is aimed to provide modes and compositions which can be useful in detection of increased/decreased inherited predisposition to cancers of various sites.

### SUMMARY OF THE INVENTION

The subject of invention is the mode of detection of genetic predisposition to cancers of various sites characterized by analysis of biological material obtained from the patient in search for combined genotypes based on C142G, G355T and C4326G SNPs within CYP1B1 gene; 1100delC, del5395, and IVS2+1G>A truncating variants and I157Tmissense variant of CHEK2 gene; and C5972T variant of BRCA2 gene, wherein the presence of any of the examined genotype combinations (presented in tables A and B) of the mentioned variants of these three genes is indicating a high-risk predisposition to breast cancer and, with high probability, colon, kidney, larynx, lung, pancreas, prostate, thyroid, female genital tract and ovaries, which is qualitatively and quantitatively different than the sum of the low-risk effects of the mentioned variants of these three genes when taken separately.

**Table A: List of CYP1B1 (at 3 codons: 48, 119 and 432), CHEK2 and BRCA2 variants that interact with each other increasing the risk of cancer development in a non-linear manner.**

| **1** | **CYP1B1** | | | **Cancer site** |
|---|---|---|---|---|
| | 48 | 119 | 432 | Breast, colon, larynx, lung, pancreas, prostate, kidney |
| 1.1 | not G/G | not G/G | C/C | |
| 1.2 | C/C | G/G | not G/G | |
| 1.3 | G/G | T/T | C/C | |
| **2** | **CHEK2** | | | Breast, colon, ovaries, prostate |
| 2.1 | 1100delC | | | |
| 2.2 | del5395 | | | |
| 2.3 | IVS2+1G>A | | | |
| 2.4 | I157T | | | |
| **3** | **BRCA2** | | | Breast |
| 3.1 | C5972T | | | |

**Table B: List of genotype combinations between different variants of CYP1B1, CHEK2 and BRCA2 (as described in table A) leading to a disproportionate increase in breast cancer risk.**

| | | |
|---|---|---|
| **CYP1B1** variants 1.1 or 1.2 or 1.3 | **CHEK2** variants 2.1 or 2.2 or 2.3 | |
| | **CHEK2** variants 2.1 or 2.2 or 2.3 or 2.4 | **BRCA2** variant 3.1 |
| **CYP1B1** variants 1.1 or 1.2 or 1.3 | **CHEK2** variants 2.1 or 2.2 or 2.3 or 2.4 | **BRCA2** variant 3.1 |

CYP1B1 germline variants at positions 142 (codon 48), 355 (codon 119) and 4326 (codon 432) as described in table A lead to the production of altered variants of the CYP1B1 RNA and protein. These variants include the following three combinations. First: 142 non-homozygous G (at least one of both alleles codes for arginine in codon 48) and 355 non-homozygous G (at least one of both alleles codes for serine in codon 119) and 4326 homozygous C (both alleles code for leucine in codon 432). Second: 142 homozygous C (both alleles code for arginine in codon 48) and 355 homozygous G (both alleles codes for alanine in codon 119) and 4326 non-homozygous G (at least one of both alleles codes for leucine in codon 432). Third: 142 homozygous G (both alleles code for glycine in codon 48) and 355 homozygous T (both alleles codes for serine in codon 119) and 4326 homozygous C (both alleles codes for leucine in codon 432). These three combinations are mutually exclusive.

CHEK2 germline variants as described in table A lead to the production of truncated, dysfunctional variant of the CHEK2 RNA and proteins in the case of the deletion of C at position 1100 in 1100delC, the deletion of 5395 bases including exons 9 and 10 in del5395 and the insertion of 4 bases due to abnormal splicing in IVS2+1G>A. CHEK2 germline variant I157T as described in table A is a common missense mutation that codes the substitution of an isoleucin for a threonin at codon 157 and leads to the production of an altered variant of the CHEK2 RNA and protein.

BRCA2 germline variant C5972T as described in table A codes for an aminoacid substitution from threonine to methionine at codon 1915 thus leading to the production of an altered variant of the BRCA2 RNA and protein.

For the needs of this invention, germline changes within CYP1B1, CHEK2 and BRCA2 genes as described above are understood as causing the production of CYP1B1, CHEK2 and BRCA2 proteins respectively with function or activity altered significantly, what is related with significantly increased risk of cancer of various sites.

We have found evidence for direct or indirect genetic interaction between the genes CYP1B1 and CHEK2, between the genes CHEK2 and BRCA2, and between the genes CYP1B1, CHEK2 and BRCA2. This invention refers to the increased risk of breast cancer development in persons with at least one of the three genotype combinations described in table B. These include: first, the presence of one of the three mutually exclusive genotypes of CYP1B1 described above (first 48non-G/G and 119non-G/G and 432C/C, second 48C/C and 119G/G and 432non-G/G, and third 48G/G and 119T/T and 432C/C) and the presence of at least one of the protein truncating variants of CHEK2 (1100delC, de15395, IVS2+1G>A); second, the presence of any of the described variants of CHEK2 (I157T, 1100delC, del5395, IVS2+1G>A) and the presence of the described BRCA2 variant (C5972T); and third the presence of one of the three mutually exclusive genotypes of CYP1B1 described above (first 4-8non-G/G and 119non-G/G and 432C/C, second 48C/C and 119G/G and 432non-G/G, and third 48G/G and 119T/T and 432C/C) the presence of any of the described variants of CHEK2 (I157T, 1100dell, del5395, IVS2+1G>A) and the presence of the described BRCA2 variant (C5972T). The increase of breast cancer risk for any of these three genotype combinations is above the expected value when adding the effects of the genetic variants of the three genes separately, thus pointing to an interaction effect which is qualitatively and quantitatively different than the sum of the effects of its components.

Occurrence of any of those three genotype combinations among cancer patients and healthy controls of the Polish population is examined as an example of application of mode according to the invention.

It can be assumed that because different CYP1B1, CHEK2 and BRCA2 variants have been reported as associated with multiorgan cancer predisposition in several populations, those variants will also be associated with significantly increased multiorgan predisposition to malignancies occurring not only among persons of Polish origin but also in other ethnic groups.

In particular realization of the mode according to the invention, the presence of the different germline changes is examined at the level of DNA, RNA or protein. The DNA can be examined for example using a technique chosen between RFLP, ASA, microarrays and sequencing.

According to the invention, it is also possible to suggest and, then, experimentally verify, other forms of CYP1B1, CHEK2 and BRCA2 DNA, RNA and protein respectively analogous to CYP1B1 forms produced as a result of A119S, R48G and L432V changes, CHEK2 forms produced as a result of I157T change and BRCA2 forms produced as a result of C5972T change.

It is recommended to include genetic alterations which are in linkage disequilibrium with the ones described here (presented in tables A and B) since their diagnostic value as positional markers may be comparable to the functional markers described here.

For other naturally occurring variants of the CYP1B1, CHEK2 or BRCA2 gene/protein, experimental verification of their involvement in carcinogenesis will be based on analysis of associations as it is described in the enclosed example.

The next subject of invention is also the composition for detection of CYP1B1, CHEK2 and BRCA2 germline changes allowing identification of persons with significantly increased genetic predisposition to breast cancer and most probably colon, kidney, larynx, lung, pancreas, prostate, thyroid, female genital tract and ovaries provided that CYP1B1 gennline variants C142G (R48G), G355T (A119S) and G4326C (V432L), CHEK2 germline variants I157T, 1100delC, del5395, IVS2+1G>A and BRCA2 germline variant C5972T (presented in tables A and B) or other variants or changes with analogous features or in linkage disequilibrium with them are studied.

The DNA, RNA and proteins can be used as material for diagnostic analyses. For diagnostic purposes it is appropriate to use also altered forms of CYP1B1, CHEK2 and BRCA2 DNA or RNA as well as proteins coded by corresponding alleles with alterations. The next subject of invention is thus the combined use of the polypeptides coded by the CYP1B1 allele containing germline variants R48G, A119S or V432L or other combinations (presented in table A), the polypeptides coded by the CHEK2 allele containing germline variants I157T, 1100delC, de15395, and/or IVS2+1G>A, the polypeptides coded by the BRCA2 allele containing germline variant C5972T, as well as other alterations in linkage disequilibrium with any of the above variants or changes with analogous features, for detection of significantly increased genetic predisposition to breast cancers, and with high probability one of the following sites: colon, kidney, larynx, lung, pancreas, prostate, thyroid female genital tract and ovaries.

According to invention the presence of polypeptides coded by CYP1B1 allele with germline alteration, CHEK2 allele with germline alteration and/or BRCA2 allele with germline alteration is detected by antibodies or other substances specific for any of those polypeptides or some of their fragments respectively.

Position of change is not determined by numbers of consecutive nucleotides or aminoacids. According to invention position of a given nucleotide or aminoacid can be marked with different numbers but has to possess the same properties.

It can be particularly valuable when antibodies or other substances specific for such polypeptide or a fragment of it recognize the epitope containing the characteristic alteration in the aminoacid sequence and in the protein structure coded by the germline variants in CYP1B1, CHEK2 and BRCA2 presented in table A.

Antibodies can be obtained, for example, by a method described by Kohler and Milstein, Nature 256 (1975)7 495, and in Meth. Enzymol. 73 (1981)9 3. Antibodies can be monoclonal, polyclonal or fragments of the antibodies (such as Fab, Fv or scFv) can be used. They can be obtained by methods described by Harlow and Lane "Antibodies, A Laboratory Manual", CSH Press, Cold Spring Harbor, 1988.

The next subject of invention is composition for detection of significantly increased genetic predisposition to tumors using techniques based on PCR and characterized by the use of at least two different primers allowing amplification of a region containing the germline variants of CYP1B1, CHEK2 and BRCA2 (presented in table A) or alterations in linkage disequilibrium with these variants or with analogous properties what allows detection of predisposition to breast cancer, and with high probability of the following sites: colon, kidney, larynx, lung, pancreas, prostate, thyroid, female genital tract and ovaries.

The next subject of invention is application of different polynucleotides containing on corresponding position 142, 355 or 4326 of exon 2/3 of human gene CYP1B1 the changes 355 G>T or 142 G>C or 4326A>G respectively or eight different oligonucleotide covering all possible genotype combinations described in table B, such as 142C 355T 4326C, 142G 355T 4326C, 142C 355G 4326C, 142C 355T 4326G, 142G 355T 4326G, 142G 355G 4326C, 142G 355G 4326G, and 142C 355G 4326G, or other changes with analogous properties or other polynucleotide coding CYP1B1 protein variant containing A119S or R48G or V432L substitution (or other with analogous properties) or polypeptides coded by above polynucleotides or antibodies specific for above polypeptides, as well as application of different polynucleotides containing nucleotide sequence changes corresponding to in CHEK2 germline variants I157T, 1100delC, del5395 and IVS2+1G>A of CHEK2 respectively or other changes with analogous properties or other polynucleotide coding CHEK2 protein variant containing I157T or other with analogous properties or containing changes with truncating properties analogous to 1100delC, duel5395 and IVS2+1G>A or polypeptides coded by above polynucleotides or antibodies specific for above polypeptides, as well as application of the polynucleotide containing nucleotide sequence changes corresponding to in BRCA2 germline variants C5972T or other with analogous properties, or polypeptides coded by above polynucleotides or antibodies specific for above polypeptides, for preparation of diagnostic composition allowing detection of significantly increased predisposition to cancer in persons carrying a specific genotype combination of those variants, namely; first, the presence of one of the three mutually exclusive genotypes of CYP1B1 described above (first 48non-G/G and 119non-G/G and 432C/C, second 48C/C and 119G/G and 432non-G/G, and third 48G/G and 119T/T and 432C/C) and the presence of at least one of the protein truncating variants of CHEK2 (1100delC, del5395, IVS2+1G>A); second, the presence of any of the described variants of CHEK2 (I157T, 1100delC, del5395, IVS2+1G>A) and the presence of the described BRCA2 variant (C5972T); and third the presence of one of the three mutually exclusive genotypes of CYP1B1 described above (first 48non-G/G and 119non-G/G and 432C/C, second 48C/C and 119G/G and 432non-G/G, and third 48G/G and 119T/T and 432C/C) the presence of any of the described variants of CHEK2 (I157T, 1100delC, del5395, IVS2+1G>A) and the presence of the described BRCA2 variant (C5972T). Above predisposition is associated with increased risk of breast cancer and probably one of the following sites: colon, kidney, larynx, lung, pancreas, prostate and, probably, thyroid, female genital tract and ovaries.

One of possible example of above polynucleotides are polynucleotides obtained using diagnostic composition for genotype combinations according to invention; examples of above polypeptides are polypeptide variants coded by the above polynucleotides and corresponding to changes presented in table A and following the genotype combinations in table B.

Definition "corresponding" applied in this description means, that position is not determined by numbers of consecutive nucleotides or aminoacid. According to invention, position of particular nucleotide or aminoacid which can be deleted or substituted, may be distinct than in wild type gene or polypeptide. Thus, "corresponding" position according to invention means that nucleotides or aminoacid may be described using different numbers, but still have the same properties. Such nucleotides or aminoacids which can be substituted or deleted are also covered by definition of "corresponding" position. Such nucleotides or aminoacids can, for example, be part of structures playing crucial role in regulation of gene expression, RNA stability or edition, as well as coding functional domains, splice sites or characteristic motifs of protein variants or its derivatives.

The next subject of invention is application of digestion enzymes which cut allele containing nucleotide (T or G) in 355 or (C or G) in 142 or (C or G) in 4326 nucleotide site of CYP1B1 gene e.g. (Eam 1105I, PdiI and OliI AvaI-Fermentas), as well as application of digestion enzymes which cut allele containing nucleotide (C or T) in 430 nucleotide and insertion IVS2+1G>A of the CHEK2 gene e.g. (PstI and Hpy188III) and the application of specific primers for multiplex PCR reaction sensitive for 1100de1C and del5395 variants of CHEK2 gene e.g. (CHK2ex10f, CHK2ex10r, CHK2delC, CHLdel2F, CHLc2R, CHLdeIR, CHLcF), as well as application of digestion enzymes which cut allele containing nucleotide (C or T) in 5972 nucleotide site of BRCA2 gene e.g. (Mph1103I Fermentas) for production of diagnostic composition allowing detection of significantly increased genetic predisposition to breast cancer and probably colon, kidney, larynx, lung, pancreas, prostate, thyroid, female genital tract and ovaries.

The next subject of invention is the mode of identification of genetic marker associated with significantly increased risk of malignancy development based upon evaluation of biological samples (DNA, RNA or protein) from patients affected by malignancy for the presence of one of the three genotype combinations described in table B; first, the presence of one of the three mutually exclusive genotypes of CYP1B1 described above (first 48non-G/G and 119non- G/G and 432C/C, second 48C/C and 119G/G and 432non-G/G, and third 48G/G and 119T/T and 432C/C) and the presence of at least one of the protein truncating variants of CHEK2 (1100delC, de15395, IVS2+1G>A); second, the presence of any of the described variants of CHEK2 (I157T, 1100delC, del5395, IVS2+1G>A) and the presence of the described BRCA2 variant (C5972T); and third the presence of one of the three mutually exclusive genotypes of CYP1B1 described above (first 48non-G/G and 119non-G/G and 432C/C, second 48C/C and 119G/G and 432non-G/G, and third 48G/G and 119T/T and 432C/C) the presence of any of the described variants of CHEK2 (I157T, 1100delC, de15395, IVS2+1G>A) and the presence of the described BRCA2 variant (C5972T), or other alterations in linkage disequilibrium with any of these variant changes. The incidence is then compared to frequency of occurrence of the corresponding genotype combination in the control population. The variant with significant overrepresentation/underrepresentation among patients with a given malignancy is considered as a genetic marker associated with significantly increased/decreased risk of malignancy development. It is valuable if the numbers of examined patients and controls are large enough and results are considered statistically significant when difference between the proportions reaches p<0.05. The effect of the different genotype combinations is compared to the effects of the germline variants of each of the genes taken separately by means of logistic regression and the effect of the combination is considered an interaction effect when exceeding the value expected by addition of the singular effects of each gene, with p<0.05. The invention will now be described by reference to the following biological examples which are merely illustrative.

### Example 1.

### Logistic regression analysis and studies of correlation between genotype combinations of germline changes in CYP1B1, CHEK2 and BRCA2 and predisposition to breast cancer. Patients

To establish the range of cancer types associated with the three genotype combinations of CYP1B1, CHEK2 and BRCA2 under study (as described in tables A and B) 1956 breast cancer patients from 8 hospitals throughout Poland (Szczecin, Bielsko-Biala, Poznań, Opole, Lublin, Kraków, Koszalin and Bydgoszcz). All cases were unselected and histopatologically confirmed. No selection criteria such as age, sex or cancer family history were used. Additionally we genotyped a control group consisting of 1173 newborns born in 2003-2004 in 8 hospitals throughout Poland (Szczecin, Bialystok, Gorzów, Katowice, Wroclaw, Poznań, Lódź i Rzeszów), 669 adults from the region of Szczecin (unselected for the occurrence of malignancies among relatives, male/female ratio 1:1) and a group of 464 women matched to age of breast cancer patients (total number of controls 2306).

### DNA isolation

5 ml peripheral blood was obtained from patients and mixed with 100 µl 1M EDTA, then was centrifuged in 50 ml polypropylene tubes by 10 minutes at 3000g in 4°C. Serum in upper faze was removed, and pellet containing cells was mixed with 45 ml buffer 2X (0,1M NH₄Cl, 0,25M KHCO₃, 1mM EDTA) and was left for 15 minutes in 4°C. Then mixture was centrifuged at 3000g for 10 minutes in 4°C. Supernatant was removed after centrifugation. The remaining pellet with leukocytes was suspended in 2X buffer and centrifuged 10 minutes at 3000g in 4°C. This purification of leukocytes in 2X buffer and centrifugation was repeated three times until pure leukocyte pellet was obtained. Then to leukocytes were mixed with 3ml digestion buffer (50mM NaCl, 25mM MgCl₂, 1mM EDTA; pH 8.0) with 200 µl 10% SDS and 500 µg Proteinase K. Digestion was carried out 24h in 37°C.

DNA was purified using phenol/chloroform. In brief digestion products was mixed with 3ml phenol buffered 0,5M Tris HCl (pH 8,4), and then 3ml chloroform and isoamyl alcohol mixture (mixed in proportion 1:25 vol/vol). Mixture was agitated for about 1 minute and centrifuged 10 minutes at 8000g in 20°C. After centrifugation upper faze was replaced to new tube, and mixed with equal volume of chloroform and thereafter centrifuged 10 minutes at 8000g. Above described purification with chloroform was repeated 3-times until protein ring in interfaze had disappeared.

The purified water faze containing DNA was mixed with 5M NaCl in proportion 10:1 (vol/vol) and 96% ethanol in the proportion of water faze with NaCl to ethanol 1:10 (vol/vol). Mixture was left overnight in 20°C. The resultant DNA pellet was placed in a new tube and purified with 70% ethanol, centrifuged at 3000g for 5 minutes, and ethanol was poured out. Then purified DNA pellet was dried in open tube for 30 minutes at 37°C. DNA resuspended in 400 µl TE buffer (25mM Tris HCl, 1mM EDTA; pH 8.4) was stored in 4°C until use.

### Restriction Fragment Length Polymorphism Polymerase Chain Reaction (RFLP-PCR)

### Variants 355T>G of CYP1B1

The 355T/T variant alteration was identified by RFLP-PCR using Eam 1105I and PdiI restriction enzyme (Fermentas). PCR was performed with primers e.g.CYP119F (CTCGTTCGCTCGCCTGGCGC) and e.g. CYP119R (GAAGTTGCGCATCATGCTGT). PCR reactions was carried out in PTC - 200 Peltier DNA ThermalCycler (MJ Research) in volume of 25 µl included: 1 µl (50ng) genomic DNA, 4 pmol each primer set, 2.5 µl PCR Buffer 2(Expand Long Template PCR System Roche - 22,5mM MgCl₂), 200 µM each dATP, dCTP, dGTP i dTTP and 1 U Taq DNA polymerase. In each reaction negative control (control without DNA) was used.

### PCR conditions:

a) Initial denaturation - 95°C 15 minutes
b) 15 cycles , each of: denaturation - 95°C 30 s
   primer annealing - 62-54,5°C 30s (decrease temperature 0,5 °C in each cycle)
   primer elongation - 72°C 2minutes
c) 21 cycles, each of:: denatution- 95°C 30 s
   primer annealing-57°C 30 s
   primer elongation - 72°C 30 s
d) elongation - 72 °C 8 minutes

Digestion was performed overnight at 37 °C in volume of 24 µl containing: 12 µl PCR product, 10 x Buffer Tango (Fermentas) and 2U Eam 1105I enzyme (Fermentas). Then, 15 µl of digestion product was mixed with 10 µl loading buffer and was electrophoresed in agarose gel (3% agarose gel (SeaKem FMC), 1X bufor TBE, 25 µg/ml ethidium bromide) at 6V/cm for 30 minutes. Separated PCR products were visualized in UV light. PCR product (250bp) was digested on two fragments: 136bp and 114bp in cases which containing nucleotide T in 355 nucleotide site of CYP1B1 gene. All cases with alterations are verified by using PdiI enzyme restriction (Fermentas). Restriction mixture in volume 18 µl containing: 4 µl PCR product, 10 x Buffer Tango (Fermentas) and 2U PdiI enzyme (Fermentas). Then, 15 µl digestion product was electophoresed in the same conditions. PCR product (250bp) was digested on two fragments: 138bp and 112bp in cases which containing nucleotide G in 355 nucleotide site of CYP1B1 gene.In addition, randomly selected cases with G/G, T/T and G/T variants were sequenced in order to confirm the presence of the A119S change. Sequencing was prepared by using conventional methods.

### Purification of PCR product

Sequencing product were placed on Microcon - 100 (Amicon) column which fit on 0,5 ml Eppendorf tube. 400 µl distillated water was added, then centrifuged for 15 minutes at 1850g in 25°C. The columns were 4 times washed with 400 µl of distillated water. After the last washing step, the columns were turned up side down and placed on a new Eppendorf tube. By centrifugation for 3 minutes at 9000g- we obtain 5 µl purified PCR product which were 4 times diluted with distillated water.

### Variants 142 G>C of CYP1B1

The variants of R48G alteration was identified by RFLP-PCR using Eco88I (AvaI) restriction enzyme (Fermentas). PCR was performed with primers e.g. F1CYP (TCCATCCAGCAGACCACGCT) and e.g. R1 (GCCGGACACCACACGGAAG). PCR reactions was carried out in PTC - 200 Peltier DNA ThermalCycler (MJ Research) in volume of 25 µl included: 1 µl (50ng) genomic DNA, 4 pmol each primer set, 2.5 µl PCR Buffer 2(Expand Long Template PCR System Roche - 22,5mM MgCl₂), 200 µM each dATP, dCTP, dGTP i dTTP and 1 U Taq DNA polymerase. In each reaction negative control (control without DNA) was used.

### PCR conditions:

b) Initial denaturation - 95°C 5 minutes
b) 29 cycles, each of: denaturation - 94°C 30 s
   primer annealing - 56 °C 30s
   primer elongation -72°C 30s
c) elongation - 72 °C 5 minutes

Digestion was performed overnight at 37 °C in volume of 24 µl containing: 12 µl PCR product, 10 x Buffer Tango (Fermentas) and 2U Eco88I (AvaI) enzyme (Fermentas). Then, 15 µl of digestion product was mixed with 10 µl loading buffer and was electrophoresed in agarose gel (4% agarose gel (SeaKem FMC), 1X bufor TBE, 25 µg/ml ethidium bromide) at 6V/cm for 30 minutes. Separated PCR products were visualized in UV light. PCR product (336bp) was digested on three fragments: 14bp, 91bp and 230bp in cases which containing nucleotide G in 142 nucleotide site of CYP1B1 gene. In addition, randomly selected cases with G/G, C/C and C/G variants were sequenced in order to confirm the presence of the R48G change. Sequencing was prepared by using conventional methods.

### Purification of PCR product

Sequencing product were placed on Microcon - 100 (Amicon) column which fit on 0,5 ml Eppendorf tube. 400 µl distillated water was added, then centrifuged for 15 minutes at 1850g in 25°C. The columns were 4 times washed with 400 µl of distillated water. After the last washing step, the columns were turned up side down and placed on a new Eppendorf tube. By centrifugation for 3 minutes at 9000g we obtain 5 µl purified PCR product which were 4 times diluted with distillated water.

### Variants 432 C>G of CYP1B1

The variants of L432V alteration was identified by RFLP-PCR using OliI restriction enzyme (Fermentas). PCR was performed with primers e.g. CYP1294F (ATGCGCTTCTCCAGCTTTGT) and e.g. CYP1294R (TATGGAGCACACCTCACCTG). PCR reactions was carried out in PTC - 200 Peltier DNA ThermalCycler (MJ Research) in volume of 25 µl included: 1 µl (50ng) genomic DNA, 4 pmol each primer set, 2.5 µl PCR Buffer 2(Expand Long Template PCR System Roche - 22,5mM MgCl₂), 200 µM each dATP, dCTP, dGTP i dTTP and 1 U Taq DNA polymerase. In each reaction negative control (control without DNA) was used.

### PCR conditions:

c) Initial denaturation - 95°C 15 minutes
b) 10 cycles , each of denaturation - 95°C 30 s
   primer annealing - 62-57°C 30s
      (decrease temperature 0,5 °C in each cycle)
   primer elongation - 72°C 2minutes
d) 30 cycles, each of:: denaturation-95°C 30 s
   primer annealing -57°C 30 s
   primer elongation - 72°C 30 s
d) elongation - 72 °C 8 minutes

Digestion was performed overnight at 37 °C in volume of 24 µl containing: 12 µl PCR product, 10 x Buffer R (Fermentas) and 2U OliI enzyme (Fermentas). Then, 15 µl of digestion product was mixed with 10 µl loading buffer and was electrophoresed in agarose gel (3% agarose gel (SeaKem FMC), 1X bufor TBE, 25 µg/ml ethidium bromide) at 6V/cm for 30 minutes. Separated PCR products were visualized in UV light. PCR product (623bp) was digested on two fragments: 132bp and 491bp in cases which containing nucleotide C in 4329 nucleotide site of CYP1B1 gene. In addition, randomly selected cases with G/G, C/C and C/G variants were sequenced in order to confirm the presence of the L432V change. Sequencing was prepared by using conventional methods.

### Purification of PCR product

Sequencing product were placed on Microcon - 100 (Amicon) column which fit on 0,5 ml Eppendorf tube. 400 µl distillated water was added, then centrifuged for 15 minutes at 1850g in 25°C. The columns were 4 times washed with 400 µl of distillated water. After the last washing step, the columns were turned up side down and placed on a new Eppendorf tube. By centrifugation for 3 minutes at 9000g we obtain 5 µl purified PCR product which were 4 times diluted with distillated water.

### Variant IVS2+1G>A of CHEK2

The IVS2+1G>A mutation was identified by RFLP-PCR using Hpy 188III (New England Biolabs). PCR was performed with primers CHEK2ex2/3F:
5'-ATTTATGAGCAATTTTTAAAC G-3' (SEQ ID NO: 35) and CHEK2ex2/3R: 5'-TCCAGTAACCATAAGATAATAATATTA C-3'(SEQ ID NO: 36).
   PCR reactions were carried out in DNA ThermalCycler 9600 (Perkin Elmer) in a volume of 25 µl included: 1 µl (50 ng) genomic DNA, 4 pmol Nbsex6f primer, 6 pmol Nbsex6r primer, 10 pmol Nbsde15 primer, 2.5 µl PCR buffer (100 mM Tris-HCl, 500 mM KCL, 15 mM MgCl2, 1 mg/ml gelatin; pH 8.6), 200 µM each dATP, dCTP, dGTP i dTTP and 1 U Taq DNA polymerase. In each reaction negative control (control without DNA) was used.

### PCR conditions:

c) Initial denaturation-95 °C. 5 minutes
b) 35 cycles, each of denaturation-94°C. 30 s
   primer annealing-53-58 °C. 45 s
   primer elongation-72°C. 60 s

Digestion was performed overnight at 37°C in volume of 20 µl containing: 5 µl PCR product, 1*NE Buffer 4 (New England Biolabs) and 2 U Hpy188III enzyme. Then, 151 µl of digestion product was mixed with 10 µl loading buffer and went electrophoresis in agarose gel (2% agarose gel (SeaKem FMC), 1* buffer TBE, 25 µg/ml ethidium bromide) at 6V/cm for 30 minutes. Separated PCR products were visualized in UV light. PCR product was digested in cases with the mutation.

### Purification of PCR product

PCR products were pipetted in Microcon-100 sample reservoir (Amicon) placed into vial, 400 µl dH2O was added to the reservoir and were centrifuged at 1850*g for 15 minutes. After centrifugation sample reservoir was inserted into a new vial, filled with 400 µl dH2O and centrifuged at 1850* g for 15 minutes. The latter was repeated 3-times. Sample reservoir was placed upside down in a new vial and then spun 3 minutes at 9000*g. All spins were carried out at 25 °C. About 5 µl of purified PCR product was seen in the vial and it was diluted in 20 µl dH2O

### Variant 430T>C of CHEK2

The 430T>C variant (Ile157Thr) was analyzed by restriction fragment length polymorphism polymerase chain reaction, using Ch157F (5'-ACCCATGTATCTA GGAGAGCTG-3' (SEQ ID NO: 37)) and Ch157R (5'-CCACTGTGATCTTCT ATGTCTGCA-3' (SEQ ID NO: 38)) primers. The reverse primer introduced artificial restriction site for PstI enzyme. The PCR products were digested in mutation positive cases. Experimental conditions were as for RFLP-PCR for the IVS2+1G>A variant of CHEK2 with exception of that 2 U PstI enzyme (instead of Hpy188III) and NE Buffer 3 (instead of NE Buffer 4) were used and RFLP-PCR products were separated in 3% agarose gel.

### Variant C5972T of BRCA2

The C5972T variant (Thr1915Met) was analyzed by restriction fragment length polymorphism PCR using b5972F (5'-CTC TCT AGA TAA TGA TGA ATG ATG CA) and b5972R (5'-CCA AAC TAA CAT CAC AAG GTG) primers. The forward primer introduces an artificial restriction site for the Mph1103I enzyme (Fermentas). PCR products were digested in mutation positive cases. Experimental conditions were as for RFLP-PCR for the IVS2+1G>A variant of CHEK2 with exception of that 2 U Mph1103I enzyme (instead of Hpyl188III) and NE Buffer 3 (instead of NE Buffer 4) were used and RFLP-PCR products were separated in 3% agarose gel and visualized in ultraviolet light.

### Multiplex Polymerase Chain Reaction

Multiplex PCR reactions was carried out in DNA ThermalCycler 9600 (Perkin Elmer) in volume of 25 µl included: 1 µl (50ng) genomic DNA, 5 pmol CHLdeIR primer, 5 pmol CHLcF primer, 5 pmol CHLdel2F primer or, respectively, 5 pmol CHLc2R primer, 5 pmol Chk2ex10f primer, 5 pmol Chk2ex10r primer, 5 pmol Chk2delC primer, 2.5 µl PCR buffer (100mM Tris-HCl, 500mM KCL, 15mM MgCl₂, 1mg/ml gelatin; pH 8.6), 200 µM each dATP, dCTP, dGTP i dTTP and 1 U Taq DNA polymerase. In each reaction negative control (control without DNA) was used.

### Variant del5395 of CHEK2

Two primers pairs were designed specifically for genotyping of a large deletion of 5395 nucleotides including exons 9 and 10 in multiplex PCR reaction. The first pair (CHLdel2F 5'-TGT AAT GAG CTG AGA TTG TGC-3'; CHLc2R 5'-CAG AAA TGA GAC AGG AAG TT-3') flanked breakpoint site in intron 8. The second pair (CHLdeIR 5'GTC TCA AAC TTG GCT GCG-3'; CHLcF 5'CTC TGT TGT GTA CAA GTG AC-3') flanked breakpoint site in intron 10. In mutation negative cases, only two PCR fragments of 379 and 522 bp were amplified from the wild type allele. The forward primer of the first pair and the reverse primer of the second pair amplified additional PCR product of 450 bp in mutation positive cases.

### PCR conditions

| | | |
|---|---|---|
| 1. | 94 OC → 10 min | |
| 2. | 94 OC → 25 sec. | 9 cycles |
| | 68 OC → 25sec. Lower tem. 1,4 OC in each cycle (68C first cycle> 55C - 9th cycle) | |
| | 72 OC → 35 sec. | |
| 3. | 94 OC → 25 sec. | |
| | 55 OC → 30 sec. | 31 cycles |
| | 72 OC → 35 sec. | |
| 4. | 4 OC → ∞ | |

### Variant 1100delC of CHEK2

The 1100delC was analyzed using an allele specific polymerase chain reaction assay using primers Chk2ex10f (5'-TTA ATT TAA GCA AAA TTA AAT GTC) Chk2ex10r (5'-GGC ATG GTG GTG TGC ATC), Chk2delC (5'-TGT AGT GCC CAA AAT CAT A). PCR conditions as for variant del5395.

### Results

The proportions in the distribution of different genotype combinations of variants of CYP1B1, CHEK2 and BRCA2 among cases and controls (as described in table A) were compared including and excluding the missense variant I157T of CHEK2, respectively. Since the protein truncating variants del5395, 1100delC and IVS2+1G>A of CHEK2 completely disrupt the function of the CHEK2 protein and the I157T missense variant just alters it, it seemed sensible to analyze the protein truncating variants also separately.

All combinations analyzed (as described in table 1) showed an increased proportion among cases in comparison with controls. The exclusion of the missense variant of CHEK2 strongly aggravated that tendency (greater odds ratios), but with generally lower numbers of affected cases and controls thus pointing to a more specific potential for identification of the risk group, however at the cost of significantly reducing the coverage of the population of patients. In the case of the genotype combinations of all variant of the three genes but excluding the CHEK2 missense variant, the estimated risk was highest (5.9-fold) but the power dropped below the significance level.

Also for the co-occurrence of the genotype combinations analyzed for the CYP1B1 variants (described in tables A and B) and the C5972T variant of BRCA2 the modest increase in the risk observed (1.3) was not statistically significant. The co-occurrence of the genotype combinations analyzed for CYP1B1 (described in tables A and B) and any of the CHEK2 variants resulted also in just a modest risk increase (1.4-fold) although this time statistically significant.

The greatest statistically significant differences were observed for the following genotype combinations: CYP1B1 (1.1 or 1.2 or 1.3) and CHEK2 (2.1 or 2.2 or 2.3) with an odds ratio of 2.8 (p=0.0012); CHEK2 (2.1 or 2.2 or 2.3) and BRCA2 (3.1) with an odds ratio of 5.3 (p=0.02); CHEK2 (2.1 or 2.2 or 2.3 or 2.4) and BRCA2 (3.1) with an odds ratio of 2.9 (p=0.0004); CYP1B1 (1.1 or 1.2 or 1.3) and CHEK2 (2.1 or 2.2 or 2.3 or 2.4) and BRCA2 (3.1) with an odds ratio of 4.7 (p=0.009).

**Table 1. Comparison of proportions in the distribution of different genotype combinations of variants of CYP1B1, CHEK2 and BRCA2 among cases and controls (as described in table A).**

| **Genotype combination** | | | Cases (+) | Total cases | Controls (+) | Total controls | OR 95%CI | p-value |
|---|---|---|---|---|---|---|---|---|
| **CYP1B1** 1.1 or 1.2 or 1.3 | **CHEK2** 2.1 or 2.2 or 2.3 | | 31 | 1956 | 13 | 2306 | 2.8 (1.5-5.4) | 0.0012 |
| **CYP1B1** 1.1 or 1.2 or 1.3 | **CHEK2** 2.1 or 2.2 or 2.3 or 2.4 | | 101 | 1956 | 85 | 2306 | 1.4 (1.1-1.9) | 0.02 |
| | **CHEK2** 2.1 or 2.2 or 2.3 | **BRCA2** 3.1 | 9 | 1956 | 2 | 2306 | 5.3 (1.1-25) | 0.02 |
| | **CHEK2** 2.1 or 2.2 or 2.3 or 2.4 | **BRCA2** 3.1 | 37 | 1956 | 15 | 2306 | 2.9 (1.6-5.4) | 0.0004 |
| **CYP1 B1** 1.1 or 1.2 or 1.3 | | **BRCA2** 3.1 | 60 | 1956 | 55 | 2306 | 1.3 | n.s. |
| **CYP1 B1** 1.1 or 1.2 or 1.3 | **CHEK2** 2.1 or 2.2 or 2.3 | **BRCA2** 3.1 | 5 | 1956 | 1 | 2306 | 5.9 | n.s. |
| **CYP1B1** 1.1 or 1.2 or 1.3 | **CHEK2** 2.1 or 2.2 or 2.3 or 2.4 | **BRCA2** 3.1 | 12 | 1956 | 3 | 2306 | 4.7 (1.3-17) | 0.009 |

The comparison of the differences between observed odds ratios and odds ratios expected for an additive model, wherein the different variants have independent effects and their co-occurrence increases the cancer risk in a simple additive manner (described in table 2) revealed crucial results. It has to be noted that the cancer predisposing effects of BRCA2 C5972T variant affects only the development of cancer at young age. In a non-selected group of consecutive breast cancer cases, as is in this example, the effect is diluted (OR=0,82). Therefore it is even more impressive to see the increase in breast cancer risk achieved for the whole group of patients in combined genotypes as described below.

These results (as described in table 1 and 2) show that interaction effects between CHEK2 and BRCA2 could be mainly due to the protein truncating variants of CHEK2, since the exclusion of the CHEK2 missense variant always increases dramatically the differences in all analyzed genotype combinations. The results also point to an interaction between CYP1B1 and CHEK2, but not to an interaction between CYP1B1 and BRCA2. Therefore, in the genotype combinations considering all three genes we should expect to find the confluence of two independent interaction effects instead of three interlaced.

Logistic regression was therefore applied to the data taking just combinations of 2 into consideration. Results show for the first time that CHEK2 and BRCA2 have a real interaction effect (p=0.038). That means that their effect on the disease risk is qualitatively and quantitatively different than the sum of their separate effects.

However, the expected interaction (table 2) between CYP1B1 and CHEK2 (just protein truncating mutations) could not be confirmed (p=0.078), although the tendency is evident and the failure in the confirmation is most probably due to insufficient total numbers of cases and controls analyzed: the numbers of affected cases and controls are just slightly lower than in the case of CHEK2 and BRCA2 (table 1). So it is reasonable to think that increasing the number of individuals analyzed it should be possible to show the interaction between CYP1B1 and CHEK2 (just protein truncating mutations) strongly suggested by the data of the distribution of their co-occurrence among cases as compared to controls.

**Table 2. Expected and observed increase in breast cancer risk depending on the presence of single mutations and their co-occurrence in genotype combinations.**

| **Genotype combination** | | | Observed OR | Expected OR |
|---|---|---|---|---|
| **CYP1B1** 1.1 or 1.2 or 1.3 | | | 1.4 | - |
| | **CHEK2** 2.1 or 2.2 or 2.3 | | 1.6 | - |
| | **CHEK2** 2.1 or 2.2 or 2.3 or 2.4 | | 1.2 | - |
| | | **BRCA2** 3.1 | 0.8 | - |
| **CYP1B1** 1.1 or 1.2 or 1.3 | **CHEK2** 2.1 or 2.2 or 2.3 | | 2.8 | 2.0 |
| **CYP1B1** 1.1 or 1.2 or 1.3 | **CHEK2** 2.1 or 2.2 or 2.3 or 2.4 | | 1.4 | 1.6 |
| | **CHEK2** 2.1 or 2.2 or 2.3 | **BRCA2** 3.1 | 5.3 | 1.4 |
| | **CHEK2** 2.1 or 2.2 or 2.3 or 2.4 | **BRCA2** 3.1 | 2.9 | 1.0 |
| **CYP1B1** 1.1 or 1.2 or 1.3 | | **BRCA2** 3.1 | 1.3 | 1.2 |
| **CYP1B1** 1.1 or 1.2 or 1.3 | **CHEK2** 2.1 or 2.2 or 2.3 | **BRCA2** 3.1 | 5.9 | 1.8 |
| **CYP1B1** 1.1 or 1.2 or 1.3 | **CHEK2** 2.1 or 2.2 or 2.3 or 2.4 | **BRCA2** 3.1 | 4.7 | 1.4 |

### Future experiments:

1. Extension of analyses to other polymorphisms of the same genes CYP1B1, CHEK2 and BRCA2.
2. Extension of analyses on all cancer sites.
3. Analyses of interactions with other genes/changes recognized in our laboratory as involved in predisposition to cancers (e.g. NOD2, p16, XPD, NBS1, etc.)

### REFERENCES:

Ahn JY, Schwarz JK, Piwnica-Worms H, Canman CE. (2000) Threonine 68 phosphorylation by ataxia telangiectasia mutated is required for efficient activation of Chk2 in response to ionizing radiation. Cancer Res. 60(21):5934-6.
Bailey,L.R., Roodi,N., Dupont, W.D. and Parl, F.F. (1998) Association of cytochrome P450 1B1 (CYP1B1) polymorphism with steroid receptor status in breast cancer. Cancer Res., 58, 5038-5041.
Chakravarti, D., Mailander, P.C., Li, K.M., Higginbotham, S., Zhang, H.L., Gross, M., Cavalieri E.L., Rogan, E.G., (2001) Evidence that a burst of DNA depurination in SENCAR mouse skin induces error-prone repair and forms mutations in the H-ras gene, Oncogene, 20, 7945-7953
Chang, B.L., Zheng S.L., Isaacs S.D., Turner A.R., Hawkins G.A., Wiley K.E., Bleecker E.R., (2003) Polymorphisms in the CYP1B1 gene are associated with increased risk of prostate cancer. British Journal of Cancer,89,1524-1529
Chaturvedi P, Eng WK, Zhu Y, Mattern MR, Mishra R, Hurle MR, Zhang X, Annan RS, Lu Q, Faucette LF, Scott GF, Li X, Carr SA, Johnson RK, Winkler JD, Zhou BB. (1999) Mammalian Chk2 is a downstream effector of the ATM-dependent DNA damage checkpoint pathway. Oncogene 18(28):4047-54
Chehab NH, Malikzay A, Appel M, Halazonetis TD. (2000) Chk2/hCds1 functions as a DNA damage checkpoint in G(1) by stabilizing p53.Genes Dev. 14(3):278-88
Cicek, M.S., Liu, X., Casey, G., Witte, J.S., (2005) Role of androgen metabolism genes CYP1B1, PSA/KLK3, and CYP11alpha in prostate cancer risk and aggressiveness.Cancer Epidemiol Biomarkers Prev.,14(9),2173-7
Cybulski C, Wokolorczyk D, Huzarski T, Byrski T, Gronwald J, Gorski B, Debniak T, Masojc B, Jakubowska A, van de Wetering T, Narod SA, Lubinski J. (2006) A deletion in CHEK2 of 5,395 bp predisposes to breast cancer in Poland. Breast Cancer Res Treat. [Epub ahead of print] PMID: 16897426
Cybulski C, Gorski B, Huzarski T, Byrski T, Gronwald J, Debniak T, Wokolorczyk D, Jakubowska A, Kowalska E, Oszurek O, Narod SA, Lubinski J. (2006) CHEK2-positive breast cancers in young Polish women. Clin Cancer Res. 12(16):4832-5.
Davies AA, Masson JY, McIlwraith MJ, Stasiak AZ, Stasiak A, Venkitaraman AR, West SC. (2001) Role of BRCA2 in control of the RAD51 recombination and DNA repair protein. Mol Cell 7(2):273-82.
Falck J, Mailand N, Syljuasen RG, Bartek J, Lukas J. (2001) The ATM-Chk2-Cdc25A checkpoint pathway guards against radioresistant DNA synthesis. Nature 410(6830):842-7. Friedman E, Bar-Sade Bruchim R, Kruglikova A, Risel S, Levy-Lahad E, Halle D, Bar-On E, Gershoni-Baruch R, Dagan E, Kepten I, Peretz T, Lerer I, Wienberg N, Shushan A, Abeliovich AD. (1998) Double heterozygotes for the Ashkenazi founder mutations in BRCA1 and BRCA2 genes. Am J Hum Genet. 63(4):1224-7
Fritsche, E., Bruning, T., Jonkmanns, C., (1999) Detection of cytochrome P4501B1 Bfr I polymorphism: genotype distribution in healthy German individuals and in patients with colorectal carcinoma. Pharmacogenetics, 9,405-408
Goodman, M.T., McDuffie, K., Kolonel, L.N., Terada K., (2001) Case-control study of ovarian cancer and polymorphism in genes involved in catecholestrogen formation and metabolism. Cancer Epidemiol Biomarkers Prev. 10(3):209-16
Gorski B, Narod SA, Lubinski J. (2005) A common missense variant in BRCA2 predisposes to early onset breast cancer. Breast Cancer Res. 7(6):R1023-7.
Hanna, I.H., Dawling, S., Roodi, N., Guengerich, F.P. and Parl, F.F. (2000) Cytochrome P450 1B1 (CYP1B1) pharmoacogenetics: association of polymorphisms with functional differences in estrogen hydroxylation activity. Cancer Res.,60,3440-3444
Hayes, C.L., Spink, D.C., Spink, B.C., Cao, J.Q., Walker, N.J., Sutter T.R., (1996) 17β-Estradiol hydroxylation catalyzed by human cytochrome P450 1B1, Proc. Natl. Acad. Sci. USA, 93,9776-9781
Healey CS, Dunning AM, Teare MD, Chase D, Parker L, Bum J, Chang-Claude J, Mannermaa A, Kataja V, Huntsman DG, Pharoah PD, Luben RN, Easton DF, Ponder BA. (2000) A common variant in BRCA2 is associated with both breast cancer risk and prenatal viability. Nat Genet. 26(3):362-4.
Jaworowska, E., Masojć, B., Tarnowska, C., Brzosko, M., Fliciński, J., Serrano- Fernandez, P., Matyjasik, J., Amernik, K., Scott, R.,J., Lubiński, J., (2006) Association between early-onset breast and laryngeal cancers. Breast Cancer Res. Treat., 97(2),215-9
Kilpivaara O, Alhopuro P, Vahteristo P, Aaltonen LA, Nevanlinna H. (2006) CHEK2 I157T associates with familial and sporadic colorectal cancer. J Med Genet. 43(7):e34.
Lee JS, Collins KM, Brown AL, Lee CH, Chung JH. (2000) hCds1-mediated phosphorylation of BRCA1 regulates the DNA damage response. Nature 404(6774):201-4.
Matsuoka S, Rotman G, Ogawa A, Shiloh Y, Tamai K, Elledge SJ. (2000) Ataxia telangiectasia-mutated phosphorylates Chk2 in vivo and in vitro. Proc Natl Acad Sci USA. 97(19):10389-94
Moynahan ME, Pierce AJ, Jasin M. (2001) BRCA2 is required for homology-directed repair of chromosomal breaks. Mol Cell 7(2):263-72.
Risch HA, McLaughlin JR, Cole DE, Rosen B, Bradley L, Kwan E, Jack E, Vesprini DJ, Kuperstein G, Abrahamson JL, Fan I, Wong B, Narod SA. (2001) Prevalence and penetrance of germline BRCA1 and BRCA2 mutations in a population series of 649 women with ovarian cancer. Am J Hum Genet. 68(3):700-10.
Rylander-Rudqvist, T.,Wedren, S., Fredrik, G., Humphreys, K., Ahlberg, S., (2003) Cytochrome P450 1B1 gene polymorphisms and post menopausal breast cancer risk. Carcinogenesis,24, 1533-1539
Sasaki, M., Tanaka, Y., Okino S.T., Nomoto, M., Yonezawa, S., (2004) Polymorphisms of the CYP1B1 gene as risk factor for human renal cell cancer. Clinical Cancer Research, 10,2015-2019
Seppala EH, Ikonen T, Mononen N, Autio V, Rokman A, Matikainen MP, Tammela TL, Schleutker J. (2003) CHEK2 variants associate with hereditary prostate cancer. Br J Cancer 89(10): 966-70.
Sharan SK, Morimatsu M, Albrecht U, Lim DS, Regel E, Dinh C, Sands A, Eichele G, Hasty P, Bradley A. Embryonic lethality and radiation hypersensitivity mediated by Rad51 in mice lacking Brca2. Nature 386(6627):804-10.
Shieh SY, Ahn J, Tamai K,Taya Y, Prives C. (2000) The human homologs of checkpoint kinases Chk1 and Cds1 (Chk2) phosphorylate p53 at multiple DNA damage-inducible sites. Genes Dev. 14(3):289-300.
Shimada, T., Hayes C.L., Yamazaki, H., Amin, S., Hecht, S.S., et al. (1996) Activation of chemically diverse procarcinogens by human cytochrome P-450 1B1. Cancer Res, 56,2979-2984
Shimada. T., Watanabe, J., Kawajiri, K., Sutter, R.T., Guengerich P.F., Gillam, M.J., Inoue, K., (1999) Catalytic properties of polymorphic human cytochrome P4501B1 variants. Carcinogenesis,20/8,1607-1613
Spink, D.C., Hayes, C.L., Young, N.R., Christou, M., Sutter, T.R., Jefcoate, C.R., Gierthy, J.F., (1994) The effects of 2,3,7,8-tetrachlorodibenzo-p-dioxin on estrogen metabolism in MCF-7 breast cancer cells: evidence for induction of a novel 17β-estradiol 4-hydroxylase. J. Steroid Biochem. Mol. Biol.,51,251-258
Spink, D.C., Spink, B.C., Cao, J.Q., Induction of cytochrome P450 1B1 and Catechol Estrogen Metbolism in ACHN Human Renal Adenocarcinoma Cells. (1997) J. Steriod. Biochem. Molec. Biol. 2/3,223-232
Szymanska-Pasternak J, Szymanska A, Medrek K, Imyanitov EN, Cybulski C, Gorski B, Magnowski P, Dziuba I, Gugala K, Debniak B, Gozdz S, Sokolenko AP, Krylova NY, Lobeiko OS, Narod SA, Lubinski J. (2006) CHEK2 variants predispose to benign, borderline and low-grade invasive ovarian tumors. Gynecol Oncol. [Epub ahead of print] PMID: 16828850
Tanaka, Y., Sasaki M., Kaneuchi, M., Shiina, H., Igawa, M., Dahiya R.,(2002) Polymorphisms of the CYP1B1 gene have higher risk for prostate cancer. BBRC,296,820-826 Watanabe, J., Shimada, T., Gillam E., Ikuto T., Suemasu K., Higashi, Y., (2000) Association of CYP1B1 genetic polymorphism with incidence to breast and lung cancer. Pharmacogenetic, 10,25-33
Thorlacius S, Olafsdottir G, Tryggvadottir L, Neuhausen S, Jonasson JG, Tavtigian SV, Tulinius H, Ogmundsdottir HM, Eyfjord JE. (1996) A single BRCA2 mutation in male and female breast cancer families from Iceland with varied cancer phenotypes. Nat Genet. 13(1):117-9.
Tonin PN, Mes-Masson AM, Futreal PA, Morgan K, Mahon M, Foulkes WD, Cole DE, Provencher D, Ghadirian P, Narod SA. (1998) Founder BRCA1 and BRCA2 mutations in French Canadian breast and ovarian cancer families. Am J Hum Genet. 63(5):1341-51.
Tulinius H, Olafsdottir GH, Sigvaldason H, Arason A, Barkardottir RB, Egilsson V, Ogmundsdottir HM, Tryggvadottir L, Gudlaugsdottir S, Eyfjord JE. (2002) The effect of a single BRCA2 mutation on cancer in Iceland. J Med Genet. 39(7):457-62.
Weischer M, Bojesen SE, Tybjaerg-Hansen A, Axelsson CK, Nordestgaard BG. (2006) Increased Risk of Breast Cancer Associated With CHEK2*1100delC. J Clin Oncol. [Epub ahead of print] PMID: 16880452
Xia F, Taghian DG, DeFrank JS, Zeng ZC, Willers H, Iliakis G, Powell-SN.(2001) Deficiency of human BRCA2 leads to impaired homologous recombination but maintains normal nonhomologous end joining. Proc Natl Acad Sci USA 98(15):8644-9

## Claims

1. A method for detecting a disproportionate increase in breast cancer risk in human subject **characterized in that** it comprises
a) identifying in a biological sample from the subject the genotypes of:
i) CYP1B1 gene variants at positions 142 (codon 48), 355 (codon 119) and 4326 (codon 432), wherein the CYP1B1 gene genotype variants comprise the following combinations:
- 142 not G/G, 355 not G/G, 4326 C/C, or
- 142C/C, 355G/G, 4326 not G/G, or
- 142G/G, 355T/T, 4326C/C;
ii) 1100delC, del5395, IVS2+1G>A truncating variants and I157T missense variant within CHEK2 gene;
iii) C5972T variant within BRCA2 gene;
b) establishing a presence of a genotype leading to a disproportionate increase in breast cancer risk in case of identification at least one of the following combined genotype:
- at least one of the CYP1B1 gene genotype variant as indicated in point i) combined with at least one of the CHEK2 gene genotype variant as indicated in point ii), or
- at least one of the CHEK2 gene genotype variant as indicated in point ii) combined with BRCA2 gene genotype variant as indicated in point iii), or
- at least one of the CYP1B1 gene genotype variant as indicated in point i) and at least one of the CHEK2 gene genotype variant as indicated in point ii) and BRCA2 gene genotype variant as indicated in point iii).

2. The method of claim 1, wherein investigated human subject is person of Polish or other ethnic origin.

3. The method of claim 1, wherein genetic testing is indicated to be performed among all adults.

4. The method of claim 1, wherein presence of genotype variant is detected by analysis of DNA, RNA or proteins.

5. The method according to claim 4, wherein DNA or RNA testing is performed with the use of any technique of indirect mutation detection, more preferably selected among ASA-, ASO-, RFLP-PCR, microarrays, or methods of direct mutation detection such as sequencing,

6. The method of claim 4, wherein the presence of the polypeptide encoded by allele within specific genotype variant is detected with the use of antibodies or other substances specific for this polypeptide or its fragment.

7. Use of the combined genotype of CYP1B1 gene, CHEK2 gene and BRCA2 gene as defined in claim 1 for in vitro diagnosing a disproportionate increase in breast cancer risk in human subject.

8. The use according to claim 7, wherein genotype variant is detected by method chosen among any technique of indirect mutation detection, preferably selected among ASA-ASO, RFPL-PCR, microarrays, or method of direct mutation detection, preferably sequencing, and the presence of the polypeptide encoded by allele specific for this genotype variant is detected with the use of antibodies or other substances specific for this polypeptide or its fragment.

## Patentansprüche

1. Verfahren zum Detektieren eines überproportionalen Anstiegs des Brustkrebsrisikos in einem humanen Subjekt, **gekennzeichnet dadurch dass** es folgendes umfasst:
a) Identifizieren der Genotypen
i) CYP1B1 Genvarianten an Positionen 142 (Codon 48), 355 (Codon 119) und 4326 (Codon 432), wobei die CYP1B1 Gen-Genotyp-Varianten die folgenden Kombinationen umfassen:
- 142 nicht G/G, 355 nicht G/G, 4326 C/C, oder
- 142 C/C, 355 G/G, 4326 nicht G/G, oder
- 142 G/G, 355 T/T, 4326 C/C;
ii) 1100delC, de15395, IVS2+1G>A Trunkierungsvarianten und I157T Missensevariante innerhalb des CHEK2 Gens;
iii) C5972T Variante innerhalb des BRCA2 Gens,
in einer biologischen Probe des Subjekts;
b) Feststellen des Vorhandenseins eines Genotyps, welcher zu einem überproportionalen Anstieg des Brustkrebsrisikos führt, im Falle der Identifikation von mindestens einem der folgenden kombinierten Genotypen:
- mindestens eine der unter Punkt i) angegebenen CYP1B1 Gen-Genotyp-Varianten in Kombination mit mindestens einer der unter Punkt ii) angegebenen CHEK2 Gen-Genotyp-Varianten , oder
- mindestens eine der unter Punkt ii) angegebenen CHEK2 Gen-Genotyp-Varianten in Kombination mit der unter Punkt iii) angegebenen BRCA2 Gen-Genotyp-Variante, oder
- mindestens eine der unter Punkt i) angegebenen CYP1 B1 Gen-Genotyp-Varianten und mindestens eine der unter Punkt ii) angegebenen CHEK2 Gen-Genotyp-Varianten und die unter Punkt iii) angegebene BRCA2 Gen-Genotyp-Variante.

2. Das Verfahren nach Anspruch 1, wobei das untersuchte humane Subjekt eine Person polnischer oder anderer ethnischer Herkunft ist.

3. Das Verfahren nach Anspruch 1, wobei angegeben wird, dass die genetischen Tests an allen Erwachsenen durchgeführt werden.

4. Das Verfahren nach Anspruch 1, wobei das Vorhandensein einer Genotypvariante durch die Analyse von DNS, RNS oder Proteinen festgestellt wird.

5. Das Verfahren nach Anspruch 4, wobei die Analyse von DNS oder RNS mit Hilfe einer Methode zur indirekten Detektion von Mutationen, vorzugsweise ausgewählt aus ASA-, ASO-, RFLP-PCR, Microarrays, oder Methoden zur direkten Detektion von Mutationen, wie Sequenzierung, durchgeführt wird.

6. Das Verfahren nach Anspruch 4, wobei die Anwesenheit des Polypeptids, welches von einem Allel einer spezifischen Genotypvariante codiert wird, mit Hilfe von Antikörpern oder anderen Substanzen, welche spezifisch für dieses Polypeptid oder dessen Fragment sind, detektiert wird.

7. Verwendung einer Kombination aus den Genotypen des CYP1 B1 Gens, des CHEK2 Gens und des BRCA2 Gens wie in Anspruch 1 definiert zur *in vitro* Diagnose eines überproportionalen Anstiegs des Brustkrebsrisikos in einem humanen Subjekt.

8. Die Verwendung nach Anspruch 7, wobei die Genotypvariante durch ein Verfahren ausgesucht aus einem Verfahren zur indirekten Detektion von Mutationen, vorzugsweise ASA-, ASO-, RFLP-PCR, Microarrays, oder einer Methode zur direkten Detektion von Mutationen, vorzugsweise Sequenzierung, detektiert wird, und die Anwesenheit des Polypeptids, welches von einem Allel, das für diese Genotypvariante spezifisch ist, codiert wird, mit Hilfe von Antikörpern oder anderen Substanzen, welche spezifisch für dieses Polypeptid oder dessen Fragment sind, detektiert wird.

## Revendications

1. Procédé de détection d'une augmentation disproportionnée du risque de cancer du sein chez un sujet humain **caractérisé en ce qu'**il comprend
a) l'identification dans un échantillon biologique provenant du sujet des génotypes de:
i) variants du gène CYP1B1 aux positions 142 (codon 48), 355 (codon 119) et 4326 (codon 432), où les variants génotypiques du gène CYP1B1 comprennent les combinaisons suivantes:
- 142 non G/G, 355 non G/G, 4326 C/C, ou
- 142 C/C, 355 G/G, 4326 non G/G, ou
- 142 G/G, 355 T/T, 4326 C/C;
ii) variants par troncature 1100delC, del5395, IVS2+1G>A et variant faux-sens I157T au sein du gène CHEK2;
iii) variant C5972T au sein du gène BRCA2;
b) l'établissement de la présence d'un génotype menant à une augmentation disproportionnée du risque de cancer du sein en cas d'identification d'au moins l'un des génotypes combinés suivants:
- au moins l'un des variants génotypiques du gène CYP1B1 tels qu'indiqués dans le point i) combiné avec au moins l'un des variants génotypiques du gène CHEK2 tels qu'indiqués dans le point ii), ou
- au moins l'un des variants génotypiques du gène CHEK2 tels qu'indiqués dans le point ii) combiné avec le variant génotypique du gène BRCA2 tel qu'indiqué dans le point iii), ou
- au moins l'un des variants génotypiques du gène CYP1B1 tels qu'indiqués dans le point i) et au moins l'un des variants génotypiques du gène CHEK2 tels qu'indiqués dans le point ii) et le variant génotypique du gène BRCA2 tel qu'indiqué dans le point iii).

2. Procédé selon la revendication 1, dans lequel le sujet humain étudié est une personne d'origine ethnique polonaise ou autre.

3. Procédé selon la revendication 1, dans lequel l'analyse génétique est indiquée pour être effectuée parmi tous les adultes.

4. Procédé selon la revendication 1, dans lequel la présence d'un variant génotypique est détectée par l'analyse d'ADN, d'ARN ou de protéines.

5. Procédé selon la revendication 4, dans lequel l'analyse d'ADN ou d'ARN est effectuée avec l'utilisation de toute technique de détection indirecte de mutations, plus préférentiellement choisie parmi la PCR ASA, ASO, RFLP, des micropuces ou des procédés de détection directe de mutations comme le séquençage.

6. Procédé selon la revendication 4, dans lequel la présence du polypeptide codé par l'allèle au sein du variant génotypique spécifique est détectée avec l'utilisation d'anticorps ou d'autres substances spécifiques de ce polypeptide ou de son fragment.

7. Utilisation du génotype combiné du gène CYP1B1, du gène CHEK2 et du gène BRCA2 tel que défini dans la revendication 1 pour un diagnostic *in vitro* d'une augmentation disproportionnée du risque de cancer du sein chez un sujet humain.

8. Utilisation selon la revendication 7, dans laquelle le variant génotypique est détecté par un procédé choisi parmi toute technique de détection indirecte de mutations, choisie de préférence parmi la PCR ASA, ASO, RFLP, des micropuces ou un procédé de détection directe de mutations, de préférence le séquençage, et la présence du polypeptide codé par l'allèle spécifique de ce variant génotypique est détectée avec l'utilisation d'anticorps ou d'autres substances spécifiques de ce polypeptide ou de son fragment.
